# FASCICULE DE BREVET EUROPEEN

(11) **EP 1 025 264 B1**
(45) Date de publication et mention de la délivrance du brevet: **14.03.2007**
(21) Numéro de dépôt: 98952816.1
(22) Date de dépôt: 29.10.1998
(51) Int. Cl.: C12Q 1/68

(54) **Méthode de diagnostique in vitro de pathologies associées à des remaniements géniques et trousses de diagnostic**
Diagnostisches Verfahren in vitro hinsichtlich durch Translokation verursachter Pathologien sowie diagnostische Testsysteme
In vitro method for diagnosing pathologies associated with gene arrangements and diagnostic kit

(30) Priorité: 30.10.1997 FR 9713656
(43) Date de publication de la demande: 09.08.2000
(73) Titulaire: IPSOGEN SAS, 13288 Marseille Cedex 09 (FR)
(72) Inventeur: Gabert, Jean, 13008 Marseille (FR)
(74) Mandataire: Peaucelle, Chantal
(86) Numéro de dépôt international: PCT/FR1998/002320
(87) Numéro de publication internationale: WO 1999/023251

(56) Documents cités:
- WO-A-93/25713
- WO-A-94/02500
- WO-A-94/13793
- WO-A-96/13514
- US-A- 5 024 934
- BARR F G ET AL.: "A consensus polymerase chain reaction - oligonucleotide hybridization approach for the detection of chromosomal translocations in pediatric bone and soft tissue sarcomas" AMERICAN JOURNAL OF CLINICAL PATHOLOGY, vol. 104, 1995, pages 627-633, XP002071719
- AKASAKA T ET AL.: "Application of long-distance polymerase chain reaction to detection of junctional sequences created by chromosomal translocation in mature B-cell neoplasms" BLOOD, vol. 88, no. 3, 1996, pages 985-994, XP002071720
- PETER M ET AL.: "An EWS/ERG fusion with a truncated N-terminal domain of EWS in a Ewing's tumor" INTERNATIONAL JOURNAL OF CANCER, vol. 67, 1996, pages 339-342, XP002071721
- RATECH H AND MASIH A: "Sensitive detection of clonal antigen receptor gene arrangements in non-hodgkin's malignant lymphoma with an anchored polymerase chain reaction-based strategy" AMERICAN JOURNAL OF CLINICAL PATHOLOGY, vol. 100, 1993, pages 527-533, XP002071722
- KEHRER-SAWATZKI H ET AL.: "The second case of a t/17;22) in a family with neurofibromatosis type 1: sequence analysis of the breakpoint regions" HUMAN GENETICS, vol. 99, 1997, pages 237-247, XP002071723
- CORRAL J ET AL.: "Acute leukemias of different lineages have similar MLL gene fusions encoding related chimeric proteins resulting from chromosomal translocation" PROCEEDINGS OF THE NATIONAL ACADEMY OF SCIENCE USA, vol. 90, 1993, pages 8538-8542, XP002071724 cité dans la demande
- GABERT J ET AL.: "Detection of MLL fusion genes by a race-based PCR screening" BLOOD, vol. 90, no. 10 Suppl. 1, 1997, page 500a XP002092468

### LISTE DE SEQUENCES

**(1) INFORMATIONS GENERALES:**
   (i) DEPOSANT:
      (A) NOM: Jean GABERT
      (B) RUE: 70 Chemin du Lancier
      (C) VILLE: MARSEILLE
      (E) PAYS: FRANCE
      (F) CODE POSTAL: 13008
   (ii) TITRE DE L'INVENTION: Méthode de diagnostic *in vitro* de pathologies associées à des remaniements géniques et trousses de diagnostic.
   (iii) NOMBRE DE SEQUENCES: 7
   (iv) FORME DECHIFFRABLE PAR ORDINATEUR:
      (A) TYPE DE SUPPORT: Floppy disk
      (B) ORDINATEUR: IBM PC compatible
      (C) SYSTEME D'EXPLOITATION: PC-DOS/MS-DOS
      (D) LOGICIEL: PatentIn Release #1.0, Version #1.30 (OEB)
**(2) INFORMATIONS POUR LA SEQ ID NO: 1:**
   (i) CARACTERISTIQUES DE LA SEQUENCE:
      (A) LONGUEUR: 52 paires de bases
      (B) TYPE: nucléotide
      (C) NOMBRE DE BRINS: simple
      (D) CONFIGURATION: linéaire
   (ii) TYPE DE MOLECULE: ADN
   (xi) DESCRIPTION DE LA SEQUENCE : SEQ ID NO: 1:
      CGTCGTCGTG AATTCCTAGA TCTTCTAGAT ATGTTTTTTT TTTTTTTTTT VV 52
**(3) INFORMATIONS POUR LA SEQ ID NO: 2:**
   (i) CARACTERISTIQUES DE LA SEQUENCE:
      (A) LONGUEUR: 44 paires de bases
      (B) TYPE: nucléotide
      (C) NOMBRE DE BRINS: simple
      (D) CONFIGURATION: linéaire
   (ii) TYPE DE MOLECULE: ADN
   (xi) DESCRIPTION DE LA SEQUENCE : SEQ ID NO: 2:
      CGTCGTCGTG AATTCCTAGA TCTTCTAGAT ATGTTNNNNN NNNN 44
**(4) INFORMATIONS POUR LA SEQ ID NO: 3:**
   (i) CARACTERISTIQUES DE LA SEQUENCE:
      (A) LONGUEUR: 32 paires de bases
      (B) TYPE: nucléotide
      (C) NOMBRE DE BRINS: simple
      (D) CONFIGURATION: linéaire
   (ii) TYPE DE MOLECULE: ADN
   (xi) DESCRIPTION DE LA SEQUENCE : SEQ ID NO: 3:
      AGCCCAAGTT TGGTGGTCGC AATATAAAGA AG 32
**(5) INFORMATIONS POUR LA SEQ ID NO: 4:**
   (i) CARACTERISTIQUES DE LA SEQUENCE:
      (A) LONGUEUR: 29 paires de bases
      (B) TYPE: nucléotide
      (C) NOMBRE DE BRINS: simple
      (D) CONFIGURATION: linéaire
   (ii) TYPE DE MOLECULE: ADN
   (xi) DESCRIPTION DE LA SEQUENCE : SEQ ID NO: 4:
      GCCGAATTCA TGCCTTCCAA AGCCTACCT 29
**(6) INFORMATIONS POUR LA SEQ ID NO: 5:**
   (i) CARACTERISTIQUES DE LA SEQUENCE:
      (A) LONGUEUR: 35 paires de bases
      (B) TYPE: nucléotide
      (C) NOMBRE DE BRINS: simple
      (D) CONFIGURATION: linéaire
   (ii) TYPE DE MOLECULE: ADN
   (xi) DESCRIPTION DE LA SEQUENCE : SEQ ID NO: 5:
      CGTCGTCGTG AATTCCTAGA TCTTCTAGAT ATGTT 35
**(7) INFORMATIONS POUR LA SEQ ID NO: 6:**
   (i) CARACTERISTIQUES DE LA SEQUENCE:
      (A) LONGUEUR: 36 paires de bases
      (B) TYPE: nucléotide
      (C) NOMBRE DE BRINS: simple
      (D) CONFIGURATION: en trèfle
   (ii) TYPE DE MOLECULE: ADN
   (xi) DESCRIPTION DE LA SEQUENCE : SEQ ID NO: 6:
      CUCCAGCUGA UGAGUCCGUG AGGACGAAAC CUUUGG 36
**(8) INFORMATIONS POUR LA SEQ ID NO: 7:**
   (i) CARACTERISTIQUES DE LA SEQUENCE:
      (A) LONGUEUR: 38 paires de bases
      (B) TYPE: nucléotide
      (C) NOMBRE DE BRINS: simple
      (D) CONFIGURATION : en trèfle
   (ii) TYPE DE MOLECULE: ADN
   (xi) DESCRIPTION DE LA SEQUENCE : SEQ ID NO: 7:
      CUGGAAUCUG AUGAGUCCGU GAGGACGAAA UUUUCUUC 38

## Description

L'invention se rapporte à la détection de remaniements géniques, avec échange de matériel génétique. Ces remaniements correspondent à la formation de gènes de fusion par accolement de la partie transloquée à une portion du génome située sur le chromosome partenaire, ou une modification de la régulation de l'expression d'un gène. Le terme gène ainsi utilisé désignera le gène impliqué dans divers remaniements alors que l'expression "partenaires de fusion" se réfèrera aux portions de génome accolées audit gène.

L'invention vise plus particulièrement une méthode et des trousses pour le diagnostic in vitro de pathologies associées à de tels remaniements.

En ce qui concerne par exemple les leucémies, on sait qu'elles sont associées aux remaniements de nombreux gènes dont certains, tels que le gène MLL, interviennent de manière récurrente.

Le gène MLL appartient à la bande 11q23 du génome humain, fréquemment impliquée dans des remaniements moléculaires, en particulier dans le cadre des leucémies aigües lymphoïdes (LAL) et également de leucémies aigües myéloïdes (LAM). Les études cytogénétiques effectuées ont permis de dénombrer à ce jour une trentaine de bandes chromosomiques partenaires différentes. Treize partenaires de fusion de MLL ont été actuellement clonés et séquencés, ce qui représente approximativement 95 % des remaniements connus.

Ces remaniements sont le plus souvent associés à de mauvais pronostics cliniques, d'où l'importance accordée à leur étude depuis quelques années.

La cytogénique, avec l'établissement du caryotype, correspond à l'une des méthodes classiquement utilisées. Cette technique a permis de montrer l'existence de remaniements avec un grand nombre de partenaires dans la région chromosomique 11q23 et d'établir la valeur pronostique de l'anomalie. Mais elle comporte de nombreux faux négatifs et son taux de réussite n'excède pas 50 à 70 %.

D'autres techniques connues comprennent le Southern blot et l'hybridation *in situ*.

Le Southern blot présente l'avantage de mettre en évidence tous les remaniements, mais reste peu exploitable par les laboratoires cliniques du fait de sa longueur et de sa lourdeur, et des contraintes de la radioactivité. En effet, un résultat en quelques semaines, au cas par cas, est indispensable pour une décision thérapeutique.

L'hybridation *in situ* (FISH) pourrait *a priori* permettre de mettre en évidence des anomalies génétiques, mais sa sensibilité n'est pas toujours suffisante du fait de la fréquence des délétions qui accompagnent souvent les translocations, l'application de cette technique peut également conduire à l'établissement de faux négatifs.

Les problèmes soulevés par ces deux types de démarche expliquent l'importance prise par l'amplification par PCR.

Cette technique permet en effet de mettre en évidence la présence d'un remaniement avec un partenaire particulier. Mais, dans sa réalisation actuelle, elle ne permet de détecter que les remaniements les plus fréquents et n'est pas applicable pour l'ensemble des partenaires, car le test deviendrait alors très lourd et consommerait beaucoup trop de matériel du patient.

Récemment, on a proposé une technique de PCR multiplex, qui permet la mise en évidence de 4 à 6 partenaires, voire plus. Il s'agit cependant d'une technique délicate, dont la difficulté augmente avec le nombre de partenaires à tester et qui, de plus, nécessite un appareillage très cher (séquenceur automatique avec plusieurs marqueurs fluorescents), ce qui limite sa diffusion à quelques laboratoires experts.

La solution apportée par l'invention repose sur la réalisation d'une PCR ancrée, c'est-à-dire avec un couple unique d'amorces, permettant d'amplifier sans discrimination tous les types de gènes de fusion impliquant le gène visé, et la révélation spécifique des seuls gènes de fusion. L'ensemble des types de gènes de fusion peut être amplifié et détecté selon un protocole simple et rapide d'exécution.

L'invention a donc pour but de fournir une méthode de diagnostic in vitro de pathologies associées aux remaniements de gènes, permettant de détecter de tels remaniements et pouvant être réalisée sur la quasi-totalité des patients.

Dans WO 96/13514, on décrit la caractérisation et l'isolement du gène *TCL*-1 associé à des anomalies.

Ce document vise à caractériser tous les gènes de la même famille *TCL*-1 et utilise à cet effet une technique PCR avec mise en oeuvre d'amorces dégénérées.

Dans J. Clin, Pathol, 1993, 100, p 527-533, Ratech et al rapportent l'intérêt d'une PCR ancrée pour détecter un large spectre de néoplasies de cellules B matures. Les amorces décrites sont spécifiques des IgH et ne sont donc pas totalement aléatoires. De plus, les produits de PCR obtenus sont clonés et séquencés, ce qui correspond à des techniques de caractérisation lourdes à mettre en oeuvre.

Dans Human genetics, 1997, 99, P 237-247, Kehrer - Sawatzki et al analysent des régions de cassure dans la gène *NF1* impliqué dans la neurofibromatose de type 1.

La technique PCR dite semi-spécifique mise en oeuvre est réalisée de manière à identifier le fragment de jonction dans la région de cassure, sa séquence étant ensuite analysée. Cet article n'enseigne donc pas d'amplification asymétrique pour amplifier l'ensemble des gènes de fusion et de révélation des seuls gènes impliqués dans le remaniement.

Dans PNAS, 1993, 90, p 3533-8542, Corral et al étudient la cassure dans MLL de différentes translocations et de délétions de 11q, avec clonage de certains partenaires du gène MLL aux fins d'identification, ce qui correspond à une technique utilisable en recherche, mais lourde à appliquer en opération de routine.

Elle vise également à fournir des trousses de diagnostic pour la mise en oeuvre de cette méthode.

La méthode de diagnostic in vitro, selon l'invention, est caractérisée en ce qu'on soumet de l'ADN d'un patient à au moins une étape de PCR ancrée, en effectuant au moins une étape d'amplification asymétrique, à l'aide d'un seul couple d'amorces formé par une amorce spécifique de l'ADN du gène susceptible d'être impliqué dans un gène de fusion et par une amorce complémentaire aléatoire, et qu'on ne révèle un tel gène que dans la mesure où il est impliqué dans ladite fusion.

On observera que ces dispositions permettent avantageusement d'amplifier toute séquence impliquant le gène considéré, quel que soit le partenaire de fusion, et même si la séquence associée au gène considéré présente une longueur importante. L'étape de révélation est au contraire spécifique et ne permet de détecter le gène considéré que dans la mesure où il est remanié avec un partenaire donné.

Les amorces utilisées dans l'étape d'amplification sont avantageusement choisies, en particulier pour l'amplification de long fragments, de manière à satisfaire les critères de longueur, Tm et de stabilité aux extrémités.

Ainsi, la longueur des amorces doit assurer une stabilité permettant l'élongation. Des amorces avantageuses comportent 25 à 40 nucléotides environ, et notamment de 30 à 35 nucléotides.

La température Tm, à laquelle la moitié de l'ADN est sous forme dénaturée, est avantageusement de l'ordre de 80°C à 86°C, et notamment voisine de 80°C. La composition en bases de la séquence est choisie de manière à satisfaire cette exigence.

De même, on prendra en compte la stabilité aux extrémités 3' et 5', l'extrémité de l'amorce qui subit l'élongation devant être moins stable que l'extrémité opposée, pour éviter l'initiation et l'élongation de produits de PCR non spécifiques. Il est également important d'éviter la formation de duplex et de boucles en 3', qui gèneraient le bon appariement des amorces à la séquence d'ADN ou d'ADNc

L'élaboration d'amorces telles que définies ci-dessus peut être aisément réalisée à l'aide de logiciels. La stratégie de PCR ancrée peut être dirigée en 3' comme défini ci-dessus, mais également en 5', une queue artificielle étant alors rajoutée à l'extrémité 5' du gène, utilisable comme amorce. On a recours avec avantage, à cet effet, à l'enzyme terminal déoxyribonucléotidyltransférase.

La révélation des gènes remaniés est effectuée par tout marquage approprié.

De manière générale, on met en contact des sondes spécifiques des séquences nucléotidiques de partenaires de fusion connus avec les produits de PCR dénaturés, marqués aux fins de révélation, dans des conditions permettant une interaction spécifique sondes-produits de PCR lorsqu'il existe une complémentarité des bases.

Les produits de PCR portent un marqueur (digoxigénine, biotine ou fluorophore par exemple) qui va permettre leur révélation. Ce marqueur est porté par un désoxynucléotide qui est incorporé aux produits PCR pendant la deuxième amplification.

Les sondes peuvent être fixées de façon covalente sur un support, tel que des plaques à 96 puits.

Cette fixation covalente peut être réalisée avantageusement par le couplage sonde biotinylée/plaque-streptavidine.

En variante, cette liaison covalente peut être réalisée à l'aide d'une sonde phosphorylée à son extrémité et un groupement carbodiimide sur la plaque ou encore une sonde modifiée par un groupement amine à une extrémité et liée par des groupements N-oxysuccinimide esters.

Une méthode satisfaisante comprend l'utilisation de la technique ELISA pour révéler spécifiquement celles des séquences nucléotidiques qui comportent le gène impliqué dans un remaniement.

On fait réagir les produits de PCR, dans lesquels on a incorporé un marqueur, avec un anticorps lui-même marqué, dirigé contre les marqueurs des produits de PCR, dans des conditions permettant une réaction de type antigène-anticorps, la révélation de la réaction antigène-anticorps, lorsqu'elle se produit, étant effectuée par détection du marqueur de l'anticorps ou d'une réaction l'impliquant.

Selon encore une autre méthode, on a recours à la technologie de PCR qui permet de détecter des produits de PCR par hybridation sonde interne-produit de PCR en solution. Il peut s'agir soit d'une sonde d'hybridation, soit d'une sonde d'hydrolyse.

On observera que la mise en oeuvre de PCR multiples, avec différents gènes, permet, en marquant ces derniers avec des marqueurs distincts les uns des autres, de révéler dans un même test plusieurs gènes remaniés impliqués dans une pathologie.

Une variante de révélation, permettant de mettre en évidence, dans un même test, de nombreux remaniements géniques sur un grand nombre de gènes, est basée sur la technique des puces à ADN et comprend l'utilisation de sondes oligonucléotidiques ou d'ADNc fixées sur un support miniaturisé. Chaque sonde ou unité d'hybridation peut être avantageusement contrôlée individuellement par un champ électrique.

Dans une autre variante, les sondes internes sont avantageusement immobilisées sur des bandelettes.

L'ADN soumis à amplification correspond avantageusement à l'ADNc, tel qu'obtenu par transcription inverse de l'ARN extrait de l'échantillon. En variante, il s'agit d'ADN génomique extrait de l'échantillon à étudier.

Les sondes internes sont immobilisées avantageusement sur des bandelettes.

Selon un mode de mise en oeuvre de l'invention, on procède à une étape de transcription inverse (RT en abrégé), avant l'amplification par PCR, afin de synthétiser une population d'ADNc à partir des ARN des cellules de l'échantillon à étudier.

Pour cette étape, on utilise avantageusement une séquence de nucléotides stable, dont le Tm est de l'ordre de 80 à 90°C.

Des séquences appropriées comprennent une cassette de 40 à 60 nucléotides environ et comportent à l'une des extrémités de 10 à 20 motifs T ou, en variante, une répétition d'un motif nucléotidique au hasard.

Selon un autre mode de mise en oeuvre de l'invention, on soumet l'ADN génomique ou l'ARN, extraits des cellules de l'échantillon à étudier, à l'action d'un composé capable d'inciser ou de bloquer spécifiquement l'ADN du gène dont on étudie la fusion. Il s'agit par exemple de PNA (acides nucléiques polypeptidiques) ou de ribozymes. On effectue ensuite les étapes de PCR, ou le cas échéant de RT-PCR avec des amorces comportant éventuellement des sites de clonage. Ensuite on fait réagir les produits obtenus avec d'une part deux sondes spécifiques du gène à étudier, une en amont de la région des points de cassure (sonde «a») et une en aval (sonde «b»), d'autre part des sondes élaborées à partir des gènes partenaires connus (sondes «c»). Une détection positive avec la sonde «a» et négative avec la sonde «b» permet de conclure au réarrangement du gène considéré et une révélation négative avec les sondes «c» à l'absence de détection d'un produit de fusion connu. Les nouveaux gènes de fusion, lorsqu'ils sont mis en évidence par le test, peuvent être secondairement clonés et séquencés par les techniques classiques.

Cette technique apporte ainsi des éléments dans la compréhension des événements moléculaires impliqués dans la transformation cellulaire.

Selon un mode avantageux de réalisation de l'invention, mis en oeuvre pour détecter des translocations impliquant le gène MLL, on synthétise par RT un pool d'ADNc à partir de l'ARN extrait de l'échantillon à étudier à l'aide d'amorces comportant une cassette d'environ 30 à 35 nucléotides, complétée par une séquence de 6 ou 9 motifs nucléotidiques au hasard, et on réalise une PCR ancrée avec, comme amorce sens spécifique, une amorce située sur l'exon 5 de MLL. Lorsqu'on a recours à un deuxième tour d'amplification, on utilise une amorce sens interne par rapport à la première, ce qui permet d'augmenter la spécificité. L'amorce aléatoire est avantageusement complémentaire de la cassette d'oligonucléotides utilisée lors de l'étape de transcription inverse.

La révélation des transcrits de fusion éventuellement présents est réalisée selon la technique ELISA et comprend l'utilisation de sondes situées à intervalles réguliers sur les partenaires de fusion considérés, de façon à couvrir la totalité des points de cassure.

Une première étape consiste à mettre en contact une sonde spécifique des partenaires de fusion connus de MLL avec les produits de PCR dénaturés, marqués par de la digoxygénine lors du deuxième tour de l'amplification, dans des conditions permettant une hybridation lorsqu'il existe une complémentarité de bases.

Dans une deuxième étape, on met ensuite en contact les produits résultants avec des anticorps anti-digoxygénine, ces anticorps étant couplés à une enzyme, capable de réagir avec son substrat en libérant un produit coloré détectable dans le cas où les anticorps sont fixés aux produits de PCR.

Des résultats satisfaisants sont obtenus en réalisant l'hybridation entre 37 à 50 °C environ pendant 2 à 4 heures.

L'interaction sondes-produits de PCR est réalisée à une température supérieure à 30°C, notamment de 35 à 65°C, en particulier de 55°C environ, pendant une durée de 0,5 à 5 h, notamment de 1 h environ.

Les conditions de lavage sont choisies de manière à obtenir un rapport signal/bruit de fond optimal.
On fait réagir le substrat de l'enzyme avec le mélange réactionnel sondes/produits de PCR dans les mêmes conditions de durée et de température, et on détecte le produit éventuellement libéré, par exemple par mesure de densité optique.

Les résultats obtenus à l'aide de cette méthode montrent que les produits de fusion des translocations considérées sont détectés aisément, avec des signaux forts. Ces résultats sont aisément interprétables par rapport aux contrôles négatifs.

Il est ainsi possible de détecter 95 % des remaniements du gène MLL.

Pour détecter de nouvelles associations MLL-gène partenaire, on soumet les ARN totaux à l'action de ribozymes spécifiques du gène MLL avant de procéder à la RT-PCR, puis on fait réagir les produits d'amplification avec une sonde correspondant à une séquence de l'exon 5 de MLL, en 3' de l'amorce utilisée, puis avec une deuxième sonde toujours spécifique du gène MLL située entre les points de cassure et le site d'action des ribozymes et enfin avec des sondes des partenaires connus. L'obtention d'un signal positif dans le premier cas et négatif avec la deuxième sonde sur le gène MLL est significatif d'un réarrangement de MLL, alors qu'un signal négatif dans la troisième étape indique qu'aucun produit de fusion connu n'a été détecté. On procède alors à la mise en évidence d'un nouveau gène de fusion.

En variante, la recherche de partenaires peut être réalisée en mettant en oeuvre les étapes de PCR, et le cas échéant de RT-PCR, décrites ci-dessus, et en révélant les produits de PCR à l'aide de puces d'ADN formées de sondes oligonucléotidiques ou d'ADNc fixées sur une surface miniaturisée.

L'invention vise également des trousses de diagnostic pour la mise en oeuvre de la méthode définie ci-dessus.

Ces trousses sont caractérisées en ce qu'elles comportent les réactifs nécessaires pour la réalisation d'au moins une PCR et du test de révélation, et le cas échéant de la transcription inverse et/ou de la réaction avec les agents capables d'inciser ou de bloquer spécifiquement le gène dont on étudie le remaniement, tels que les ribozymes ou les PNA.

En particulier, ces trousses comportent les amorces pour ces différentes réactions et avantageusement les solvants ou tampons appropriés pour leur réalisation, notamment pour l'hybridation et les lavages, ainsi qu'une notice d'utilisation.

Des trousses préférées renferment les sondes spécifiques de partenaires de fusion fixées à un support. Ces sondes sont par exemple fixées sur une plaque et sont telles qu'obtenues par couplage d'un réactif qu'elles comportent à l'une de leurs extrémités avec un réactif de la plaque. Il s'agit par exemple de sondes biotinylées en 5' fixées sur de la streptavidine recouvrant le fonds des puits d'une microplaque.

En variante, on utilise des sondes oligonucléotidiques ou d'ADNc fixées sur un support miniaturisé (puces à ADN).

Selon encore une autre variante, les sondes internes sont fixées sur des bandelettes.

La possibilité de stocker ces plaques-support sur lesquelles sont fixées les sondes permet de standardiser la technique de révélation et de l'alléger pour la révélation des gènes de fusion ou des transcrits de fusion recherchés.

Les expérimentations menées sur des lignées cellulaires ont été validées chez des patients dont le type de remaniement génétique avait déjà été établi, confirmant leur intérêt pour une utilisation dans un cadre clinique pour l'établissement d'un diagnostic moléculaire ainsi que pour le définition des points de cassure.

On mesurera l'intérêt particulier de la méthode de l'invention dans les cas, notamment de LAM, où l'étude cytogénétique ne révèle pas d'anomalies chromosomiques, alors que la biologie moléculaire permet de mettre en oeuvre un remaniement moléculaire. La méthode de l'invention permet ainsi de cribler les patients atteints de LAM et pour lesquels le caryotype n'est pas disponible ou a été décrit comme normal, et de vérifier l'existence ou non de remaniements associés à une pathologie.

L'invention trouve donc un intérêt particulier pour le diagnostic des leucémies.

Elle s'avère également tout spécialement utile en cancérologie. On citera en particulier le diagnostic de tumeurs solides, et tout spécialement de remaniements du gène EWS dans les tumeurs d'Ewing. L'application de la méthode de l'invention permet de détecter des remaniements EWS/FLI1 ou d'autres membres de la famille du gène ETS, tels que les gènes ERG, ETV1 ou E1AF.

De manière générale, l'invention fournit ainsi les moyens pour un diagnostic simple, fiable et de grande sensibilité, sur un grand nombre d'échantillons. L'amplification du matériel de départ revêt également un grand intérêt puisqu'il s'agit de prélèvements effectués sur les patients, notamment sang ou moelle osseuse. On peut tester un grand nombre de sondes, par exemple, jusqu'à 500 sondes environ sur les plaques 96 puits.

On notera avec intérêt que les dispositions de l'invention permettent une automatisation des tests, notamment à l'étape de révélation.

De plus, comme déjà souligné, l'invention fournit les moyens de détecter des gènes qui jusqu'alors n'avaient pas été identifiés comme impliqués dans une pathologie donnée.

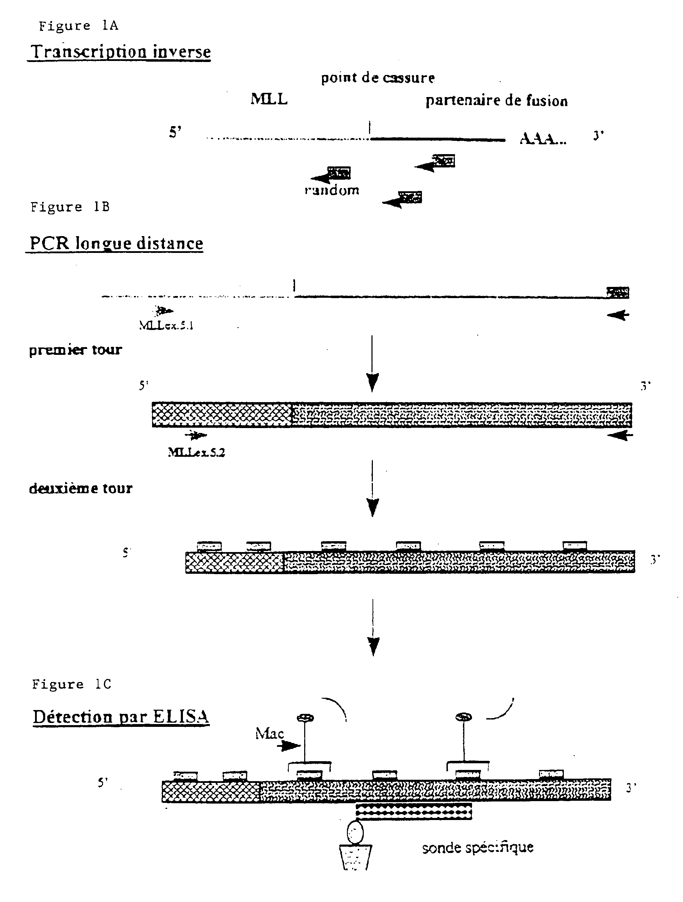

D'autres caractéristiques et avantages de l'invention seront donnés dans les exemples qui suivent.et en se référant à la figure 1A à 1C qui donne le schéma général des étapes mises en oeuvre pour une détection de transcrits de fusion.

Exemple 1 : Protocole pour la détection de remaniements d'un gène avec des partenaires de fusion connus.

A partir des ARN totaux de l'échantillon à étudier, 1) on synthétise les ADNc par transcription inverse (RT), puis 2) on amplifie le pool d'ADNc par PCR, et 3) on procède à la vérification de la spécificité des transcrits. Ces étapes sont illustrées par le schéma donné sur la figure unique.

### Préparation des ARN

Les cellules de l'échantillon à étudier sont mises en solution de lyse par addition de Trizol^{R} (Life Technologie). On ajoute ensuite du chloroforme (20% final) au lysat cellulaire obtenu, puis après 5 min d'incubation à température ambiante, le tout est centrifugé 15 min à 4°C et 12000 g.

On obtient trois phases, à savoir une phase incolore aqueuse renfermant l'ARN, une phase intermédiaire blanchâtre renfermant l'ADN, et une phase organique phénolée rouge.

On ajoute de l'isopropanol à l'ARN, (500 *µ*l pour 1 ml de Trizol^{R}), puis on centrifuge 10 min à 4°C et 12000 g, après une incubation de 10 min à température ambiante ; le précipité est rincé dans 1 ml d'éthanol 75% (5 min à 4°C et 7500 g).

Le précipité est séché à température ambiante avant d'être repris dans 10 *µ*l d'eau et traité par la RNase H.

La quantité d'ARN extrait est calculée par mesure de la DO à 260 nm : concentration (*µ*g/*µ*l) = DO mesurée x 40 (coefficient d'extinction) x coefficient de dilution x 10⁻³.

### 1). Transcription inverse

On utilise, comme système enzymatique Superscript^{R} (Life Technologie, 18064-014) ou Expand Reverse Transcrip-tase^{R} (Boehringer, 1 785 834), en appliquant le protocole suivant :

1 *µ*g d'ARN est soumis à dénaturation (volume de 9,5 *µ*l) 10 min à 70°C, puis ajouté au mélange réactionnel (10,5 *µ*l): nucléotides (1 mM) + dTT (10mM) + amorce 0,5 *µ*M) + inhibiteurs des RNases (20 unités) + enzyme (50 unités d'Expand Reverse Transcriptase^{R}, 200 unités de Superscript)^{R}, le tout dans un tampon approprié à chacun des deux systèmes enzymatiques :
Superscript^{R} : 20 mM Tris HCl, 50 mM KCl, 5 mM MgCl₂
Expand Reverse Transcriptase^{R} : 50 mM Tris HCl, 40 mM KCl, 5 mM MgCl₂.
La synthèse des ADNc se fait selon le cycle : 10 min à 20°C / 45 min à 42°C / 3 min à 99°C.

Les échantillons s.ont ensuite soumis à la RNase H (Boehringer, 786 357 : 2 unités) pendant 10 min, à 42°C.

Les ADNc sont repris dans un volume final de 60 µl (dilution au 1/3) et stockés à -20°C.

### 2). Amplification des ADNc par PCR

On rapporte les résultats obtenus avec, comme systèmes enzymatiques, ELONGASE^{R} (BRL, 10481-018) et Expand Long template PCR System (Boehringer, 175 9060).

On utilise deux programmes d'amplification différents selon la longueur des fragments :
- pour l'amplification de fragments allant jusqu'à 1 kb:

| | | | |
|---|---|---|---|
| 94°C | 3 min | | |
| 94°C | 30 sec | \| | |
| 58°C | 30 sec | \| | x 34 |
| 72°C | 30 sec | \| | |
| 16°C | ∞ | | |

- pour l'amplification de fragments supérieurs à lkb

| | | |
|---|---|---|
| 95°C | 30 sec | |
| 94°C | 10 sec / 68°C | 8 min x 10 |
| 94°C | 10 sec / 68°C | 8 min + 20 sec par cycle x20 |
| 68°C | 7 min | |
| 16°C | ∞ | |

La réaction s'effectue dans chaque cas dans un volume de 50 *µ*l en respectant les conditions suivantes : dXTP (500 *µ*M), amorces sens et antisens (1 *µ*M), MgCl₂ (3 mM), enzymes (2,5 unités), le tout dans un tampon 50 mM Tris HCl (pH 9,2), 16 mM (NH₄)₂SO₄, 2% DMSO, 0,1% Tween 20.

Un deuxième tour d'amplification est effectué lors de l'étude de longs fragments, à partir d'1 *µ*l de produit de la première PCR. On utilise alors une amorce sens interne par rapport à celle du premier tour, l'amorce antisens est identique ; en vue de la révélation par ELISA, le dTTP est remplacé par un mélange dTTP + DIG-dUTP^{R} (Boehringer ; 1558 706) respectant les proportions 1 :19.

### 3). Révélation par ELISA (kit Boehringer, 1636 111) des hybrides sondes/produits de PCR.

Dans une première étape, on met en contact des sondes biotinylées spécifiques de partenaires connus avec les produits de PCR et dans une deuxième étape on révèle les hybrides formés.

### a. hybridation

A l'aide du logiciel oligo 5, on choisit les sondes sur les séquences des différents partenaires, juste en aval des points de cassure décrits dans chacune des translocations. Les sondes sont alors biotinylées en 5' et purifiées par HPLC.
- *fixation extemporanée des sondes sur les plaques ELISA*
   10 *µ*l de produits de PCR sont dénaturés dans 10 *µ*l de solution alcaline (NaOH 0,3M), puis déposés dans un puits avec un revêtement de streptavidine, en présence de la sonde biotinylée à 7,5 pmol/ml (volume final de 220 µl). La réaction d'hybridation se fait entre 37 et 50°C, pendant trois heures et sous agitation.

Après trois lavages, l'anticorps anti-DIG couplé à la peroxydase est ajouté (2 mU dans un volume de 200 µl) ; l'incubation est de 30 min à 37°C. On procède ensuite à une série de trois lavages.

Enfin, le substrat de la peroxydase est à son tour ajouté à raison de 1 mg/ml (30 min à 37°C). La DO est lue à 405 nm contre 492 nm.
- *fixation préalable des sondes biotinylées aux plaques ELISA* (d'après R. Giorda et col., 14) :

100 *µ*l (par puits) de solution de sonde à 0,75 pmol/*µ*l sont incubés 1 heure à température ambiante, sous agitation.

Après lavage, 100*µ*l de solution 5x Denhardt's /0,02% Na azide sont déposés dans chacun des puits.

Les plaques peuvent ainsi être stockées à 4°C et utilisées au fur et à mesure des manipulations : il suffit de les laver (trois fois), puis de déposer les produits de PCR dénaturés dans 100*µ*l de tampon d'hybridation ; la suite du protocole se fait comme décrit ci-dessus.

Exemple 2 : Protocole pour la détection de remaniements d'un gène avec des partenaires de fusion inconnus.

On traite tout d'abord les ARN totaux extraits des cellules de l'échantillon à étudier par les ribosomes en opérant comme suit : 2 *µ*g d'ARN sont mis en présence des ribozymes (1 *µ*M) dans un tampon: MgCl₂ (20 mM) ; Tris HCl pH 8 (50 mM), le tout dans un volume de 10 *µ*l; le mélange réactionnel est incubé 2 heures à 37°C.

Les produits de réaction sont récupérés par précipitation avec de l'alcool absolu (2,5 volumes), en présence de glycogène et d'acétate de sodium (0,3 M final) : 30 min dans la glace puis 30 min à 14000 g, 4°C; après rinçage avec de l'alcool 75 % (20 min à 14000 g, 4°C) les précipités sont repris dans 10 *µ*l d'eau et soumis aux réactions de RT et de PCR selon l'invention.

### Exemple 3 : Détection de remaniements du gène MLL

### translocations de MLL

On rappelle dans le tableau suivant la caractérisation des translocations impliquant MLL et des partenaires de fusion telle qu'établie à ce jour.

**Tableau**

| **Transcrits de fusion de MLL** | | | | |
|---|---|---|---|---|
| **Anomalie cytogénétique** | **Partenaire de fusion** | **Famille protéique** | **Type de leucémie** | **Référence** |
| t(10;11)(p12;q23) | *ABI-1* | mABL-intermédiaire 1 | LAM | Taki T. *et al*.-1998; (1) |
| t(1;11)(p32;q23) | AF-1p=eps 15 | substrat de EGF Rct | LAM | Bernard O. *et al.-* 1994 ; (2) |
| t(1;11)(q21;q23) | AF-1q | cytokine ? | LAM | Tse W. *et al.*-1995 ; (3) |
| t(6;11)(q27;q23)* | AF6 | myosine/GLGF | T-ALL ou LAM | Prasad R. *et al.* -1993 ; (4) |
| t(11;19)(q23;p13)* | EEN | domaine SH3 | LAM | So C. *et al.* -1997 ; (5) |
| t(5;11)(q31;q23) | AF-5 | domaine GAP + NSL | CMML enfants | Berkhardt A. -1997r ; (6) |
| t(6;11)*(q21;q23') | AF6 q21 | forkhead | MDS/LAM | Bernard O. *et al.* -1997 ; (7) |
| t(X;11)(q13;q23) | AFX1 | forkhead | LAM | Corral J. *et al.* -1993 ; (8) |
| t(4;11)(q21;q23)* | AF4 | riche en ser., pro. | ALL | Gu Y. *et al.* -1992 ; (9) |
| t(9;11)(p22;q23) | AF9 | riche en ser., pro. | LAM | Nakamura T. *et al*.-1993; (10) |
| t(11;19)(q23;p13) | ENL | riche en ser., pro. | ALL | Tkachuk D. *et al.* -1992 ; (11) |
| t(10;11)(p12;q23) | AF10 | doigt de zinc + LZ | LAM | Chaplin T. *et al.* 1995; (12) |
| t(11;17)(q23;q21) | AF17 | doigt de zinc + LZ | LAM | Prasad R. *et al.* -1994 ; (13) |
| t(11;16)(q23;p13) | CBP | adaptateur transcriptionel | LAM secondaire | Taki T. *et al.* -1997 ; (14) |
| t(11;22)(q23;q13) | p300 | adaptateur transcriptionel | LAM secondaire | Ida K. *et al.* -1997 ; (15) |
| t(11;19)(d23;p13.1) | ELL | fact. d'élongation ARN pol. II | LAM | Thirman M. *et al.* - 1994 ; (16) |
| t(11;22)(q23;q11,2) | hCDCrel | cycle de division cellulaire | LAM | Megonigal M. *et al.* -1998 ; (17) |
| (+11)* | MLL | antagoniste de polycomb | LAM | Bernard O. *et al.* - 1995 ; (18) |
| Aucune | MLL | antagoniste de polycomb | LAM | Schichman S. *et al.* -1994 ; (19) |
| t(11;15)(q23;q15) | AF-15 | aucune homologie | T-ALL | Kuefer M. *et al.* -1997r ; (20) |

| | | | | |
|---|---|---|---|---|
| * = le transcrit de fusion peut être présent avec un caryotype normal. r = résumé de congrès. | | | | |

Les différents travaux menés sur MLL et ses partenaires ont permis d'établir les faits suivants :
1. Seule la protéine chimère obtenue par fusion de la partie NH₂ de MLL avec l'extrémité C terminale du partenaire semble avoir un rôle dans la tumorogénèse.
2. Malgré l'hétérogénéité des points de cassure de MLL, ceux-ci sont tous répartis entre l'exon 5 et l'exon 11 du gène ; les protéines de fusion formées sont alors homologues pour leur partie NH₂, du fait de la conservation de motifs propres à MLL.
3. Il existe un très grand nombre de partenaires : au moins autant que pour les Ig ou les TCR dans le cadre des LAL, les partenaires connus représentant plus de 95 % des translocations de MLL rapportées à ce jour. Ces partenaires ne présentent pas de réelle homologie structurale ; seuls AF9, AF4 et ENL sont homologues et AF10 et AF17 semblent appartenir à une nouvelle famille génique. MLL peut se trouver associé à lui-même dans un processus de duplication.

### . étude de cellules LAM

On rapporte ci-après les résultats obtenus avec des cellules de lignées leucémiques (LAM humaine) dont l'analyse cytogénétique révèle en particulier des réarrangements 11q23/6q27. La lignée peut donc servir de contrôle positif pour la translocation t(6 ;11).

Il s'agit de cellules de la lignée ML-2 (DSM ACC15) qui ont été cultivées en milieu RPMI (90%) + SVF (10%). En fin de culture, les cellules ont été congelées dans DMSO (5.10⁶/ml) pour être stockées, ou mises en solution de lyse (Trizol^{R}) en vue d'en extraire les acides nucléiques.

La lignée TF1 (dérivée d'un patient érythroleucémique sans anomalie 11q23) a été utilisée en tant que contrôle, à différentes étapes des manipulations (T. Kitamura et al, 21).

Les ARN sont extraits des cellules en opérant comme indiqué ci-dessus et soumis à l'action de la RNase H.

### . transcription inverse

On opère comme décrit dans l'exemple 1 en utilisant une amorce avec une répétition de 9 motifs au hasard, de séquence : 5'
CGTCGTCGTG AATTCCTAGA TCTTCTAGAT ATGTTNNNNN NNNN
(SEQ ID N° 2, 44 nuclétotides, Tm = 84°C).

### . amplification

L'amplification est réalisée comme décrit dans l'exemple 1, en utilisant comme amorces sens
- au premier tour, une amorce de séquence
   AGCCCAAGTT TGGTGGTCGC AATATAAAGA AG
   (SEQ ID N° 3, 32 nucléotides, Tm = 84°C), et
- au deuxième tour, une amorce interne de séquence
   GCCGAATTCA TGCCTTCCAA AGCCTACCT
   (SEQ ID N° 4, 29 nucléotides, Tm = 86°C), et
   comme amorce aléatoire, une amorce de séquence
   CGTCGTCGTG AATTCCTAGA TCTTCTAGAT ATGTT
   (SEQ ID N° 5, 35 nucléotides, Tm = 81°C).

### . révélation

### - hybridation

Pour chacun des partenaires de MLL, une sonde spécifique a été définie, en aval du point de cassure du contrôle positif correspondant. Le rapport signal/bruit de fond obtenu lors de l'ELISA traduit l'efficacité de révélation de chacune des sondes. Pour ENL et la duplication, la première valeur du rapport correspond à un lavage avec la solution non diluée alors que la seconde a été obtenue avec une solution diluée au 1/2.

Les sondes biotinylées utilisées sont caractérisées par un Tm compris entre 71°C et 75°C, calculé selon la méthode de la richesse en GC à l'aide d'un logiciel.

### - mesure de la DO

On mesure les DO pour chaque transcrit de fusion. Les résultats obtenus montrent que les différents partenaires sont détectés. L'obtention de signaux forts permet d'interpréter aisément les résultats par rapport aux contrôles négatifs.

De plus l'utilisation de sondes définies en aval des points de cassures rapportés dans la littérature permet de situer l'évènement moléculaire sur le gène impliqué.

### - détection de nouveaux partenaires

On soumet les ARN du contrôle positif de la t(9;11) lignée Monomac 6) et ceux de la lignée TF1 à l'action de ribozymes.

On utilise deux ribozymes dont l'action enzymatique est spécifique du gène MLL non modifié, leurs sites de coupure se situant en aval de la région des points de cassure. Ces ribozymes présentent respectivement les séquences suivantes :
- ribozyme 1 : CUCCAGCUGA UGAGUCCGUG AGGACGAAAC CUUUGG (SEQ ID N° 6)
- ribozyme 2 . CUGGAAUCUG AUGAGUCCGU GAGGACGAAA UUUUCUUC (SEQ ID N° 7).

Les séquences soulignées correspondent aux séquences complémentaires de celles de MLL autour des points de clivage, et les séquences non soulignées aux séquences non appariées aboutissant à la formation de structures secondaires indispensables à l'activité catalytique des ribozymes. Le clivage se fait en 3' du nucléotide qui suit l'uracile complémentaire de l'adénine soulignée.

Les produits de réactions ont été convertis en ADNc, puis soumis à l'amplification à l'aide d'un couple d'amorces situées de part et d'autre des points de coupure.

L'invention fournit ainsi des outils moléculaires et une méthode de diagnostic permettant d'identifier sur un grand nombre de patients les différents partenaires de MLL ainsi que leurs différents points de cassure et de mieux appréhender les mécanismes de développement de pathologies liées aux remaniements de gène, par exemple les mécanismes de leucémogénèse sous-jacents aux remaniements du gène MLL.

### BIBLIOGRAPHIE

1. Taki T. *et al,* Blood 1998, Aug 15 ; 92(4): 1125-30 Bernard O et al 1997
2. Bernard O.A. *et al,* Oncogene 1994 ;-9:1039-1045.
3. Tse W. *et al,* Blood 1995 ; 85:650-656.
4. Prasad R. *et al,* Cancer research 1993; 53; 5624-5628.
5. So C.W. *et al,* USA 1997; P.N.A.S. 94: 2563-2568.
6. Borkhardt A. *et al,* Abstract 2170, december 8, 1997. Poster Session: Tumor Suppressor genes and anti-oncogènes.
7. Bernard O.A. *et al,* Gene Chromosomes Cancer, 1998, jul. 22(3): 221-4
8. Corral J. *et al,* P.N.A.S., USA, 1993;90:8538-8542.
9. Gu Y. *et al,* Cell 1992; 71:701-708.
10. Nakamura T. *et al,* P.N.A.S. USA, 1993; 90 4631-4635.
11. Tkachuk D.C. *et al,* Cell 1992 ; 71:691-700.
12. Chaplin T. *et al,* Blood 1995 ; 85 : 1435-1441.
13. Prasad R. *et al,* P.N.A.S. USA, 1994; 91: 8107-8111.
14. Taki T. *et al,* 1997, Blood 1997, vol. 89, n° 11, pp 3945-3950.
15. Ida K. Blood 1997, Dec 15, 90(12) : 4699-704
16. Thirman M.J. *et al,* P.N.A.S. USA, 1994 ; 91:12110-12114.
17. Megonigal M.D. et al, P.N.A.S., USA, 1998 ; 95 : 6413-6418.
18. Bernard O.A. *et al*, Leukemia, 1995, vol.9:1487-1490,
19. Schichman S. *et al*, 1994, Cancer Research, vol.54, 4277-4280.
20. Kuefer M. et al, Abstract 1419,. december 8, 1997, Poster Session : Chromosome Reamangements
21. Kitamura T. et al, J Cell Physiol 1988 ; 140:323-334.

## Revendications

1. Méthode de diagnostic in vitro de pathologies associées à des remaniements géniques, comprenant une étape de PCR ancrée, **caractérisée en ce qu'**elle comprend, en association,
. une ou plusieurs étapes de PCR asymétriques, réalisée de manière non spécifiques des remaniements géniques, en utilisant un seul couple d'amorces formé par une amorce spécifique de la séquence nucléotidique correspondant au gène susceptible d'être impliqué dans un gène de fusion et par une amorce complémentaire aléatoire (amorce ancrée), et
. une étape de révélation appliquée au produit de la PCR, réalisée à l'aide de marqueurs spécifiques des remaniements géniques, afin de ne révéler que les gènes impliqués dans un remaniement, et ce dans leur totalité.

2. Méthode selon la revendication 1, **caractérisée en ce que** les amorces utilisées dans l'étape d'amplification comportent 25 à 40 nucléotides.

3. Méthode selon la revendication 1 ou 2, **caractérisée en ce que** les produits PCR sont marqués en vue de l'étape de révélation et dénaturés, puis mis en contact avec des sondes spécifiques des séquences nucléotidiques des partenaires de fusion.

4. Méthode selon la revendication 3, **caractérisée en ce que** les sondes sont fixées sur un support de manière covalente.

5. Méthode selon la revendication 3, **caractérisée en ce que** l'étape de révélation des produits de PCR est basée sur la technique des puces à ADN et comprend l'utilisation de sondes oligonucléotidiques ou d'ADNc fixées sur un support miniaturisé.

6. Méthode selon la revendication 1 ou 2, **caractérisée en ce que** les produits de PCR sont dénaturés et mis en contact avec des sondes marquées en solution.

7. Méthode selon l'une quelconque des revendications 1 à 6, **caractérisée en ce que** pour l'obtention de l'ADNc on utilise comme amorces des séquences comprenant une cassette de 40 à 60 nucléotides environ et à l'une des extrémités de 10 à 20 motifs T ou une répétition d'un motif nucléotidique au hasard.

8. Méthode selon l'une quelconque des revendications 1 à 7, **caractérisée en ce qu'**elle comprend les étapes suivantes :
- on soumet l'ADN génomique, ou l'ARN, extraits des cellules de l'échantillon à étudier, à l'action d'un composé capable d'inciser ou de bloquer spécifiquement l'ADN ou l'ARN du gène dont on étudie la fusion,
- on effectue la ou les étapes de PCR et on fait réagir les produits obtenus avec d'une part deux sondes spécifiques du gène à étudier, une en amont de la région des points de cassure et une en aval, d'autre part des sondes élaborées à partir des gènes partenaires connus, une détection positive avec la sonde d'amont et négative avec la sonde d'aval dans le premier cas permettant de conclure au réarrangement du gène considéré, et une conclusion négative dans le deuxième cas, à l'absence de détection d'un produit de fusion connu, ou qu'en variante,
- on fait réagir les produits de PCR avec une pluralité de sondes sur un support miniaturisé, l'hybridation spécifique sonde-produits de PCR marqués, mettant en évidence le partenaire du gène de fusion.

9. Méthode selon l'une quelconque des revendications 1 à 8, **caractérisée en ce qu'**elle est appliquée au diagnostic de leucémies.

10. Méthode selon l'une quelconque des revendications 1 à 8, **caractérisée en ce qu'**elle est appliquée à la détection de translocations impliquant le gène MLL et comprend :
(a) la synthèse par RT d'un pool d'ADNc à partir de l'ARN extrait de l'échantillon à étudier, en utilisant des amorces comportant une cassette d'environ 30 à 35 nucléotides complétée par une séquence de 6 ou 9 motifs nucléotidiques au hasard,
l'amplification par PCR à l'aide d'une première amorce située sur l'exon 5 de MLL, comme amorce sens spécifique,
l'amorce en 3' étant complémentaire de la séquence nucléotides connue utilisée dans l'étape de RT, suivie le cas échéant d'une nouvelle amplification par PCR, en utilisant une amorce sens interne par rapport à la première amorce.

11. Méthode selon la revendication 8, **caractérisée en ce que** la révélation des transcrits de fusion éventuellement présents comprend la mise en contact d'une sonde spécifique des partenaires de fusion connus de MLL avec les produits de PCR dénaturés, marqués par de la biotine lors de l'amplification, dans des conditions permettant une hybridation lorsqu'il existe une complémentarité de bases, la sonde étant fixée de façon covalente sur un support par couplage avec de la streptavidine.

12. Méthode selon la revendication 11, **caractérisée en ce que** pour détecter de nouvelles associations MLL-gène partenaire, on soumet les ARN totaux extraits du patient à l'action de ribozymes spécifiques du gène MLL avant de procéder à la RT-PCR, puis on fait réagir les produits d'amplification d'une part avec une sonde correspondant à une séquence de l'exon 5 de MLL, en 3' de l'amorce utilisée, puis avec une deuxième sonde toujours spécifique du gène MLL, située entre les points de cassure et le site d'action des ribozymes, et enfin avec des sondes de partenaires connus.

13. Application de la méthode selon l'une quelconque des revendications 1 à 8, au diagnostic de tumeurs solides.

14. Application de la méthode selon la revendication 13 à la détection de remaniement du gène EWS dans les tumeurs d'Ewing.

15. Application de la méthode selon la revendication 13, à la détection de remaniements des gènes ERG, ETV1 ou E1AF.

16. Trousses pour le diagnostic *in vitro* de pathologies associées à des remaniements géniques pour la mise en oeuvre de la méthode selon l'une quelconque des revendications 1 à 12, **caractérisées en ce qu'**elles comportent un couple d'amorces dont une amorce complémentaire de la séquence de nucléotides du gène cible et qui se lie à ce gène pour former un complexe, et une amorce complémentaire aléatoire, ainsi qu'au moins une sonde spécifique du partenaire de fusion, cette sonde étant liée à un support solide.

17. Trousses selon la revendication 16, **caractérisée en ce qu'**au moins une sonde biotinylée est liée à un support solide par de la streptavidine couplée au support.

18. Trousse selon la revendication 17, **caractérisée en ce que** le support solide est un support miniaturisé.

19. Trousses selon la revendication 18, **caractérisées en ce que** le support est une puce à ADN.

## Claims

1. An in vitro diagnostic method of pathologies associated with gene rearrangement, comprising a step of anchored PCR, comprising, in combination,
- one or several steps of asymmetrical PCR, carried out in a non specific manner, of gene rearrangements, by using a single pair of primers consisting in one primer specific of the nucleotidic sequence corresponding to the gene liable to be involved in a fusion gene and one random complementary primer (anchored primer), and
- a detection step applied to the PCR product, carried out by means of markers specific of gene rearrangements, in order to only detect the genes involved in a rearrangement, in their whole.

2. The method according to claim 1, wherein the primers used in the amplification step include 25 to 40 nucleotides.

3. The method according to claim 1 or 2, wherein the PCR products are marked in view of the detection step and are denatured, then put into contact with probes specific of the nucleotidic sequences of the fusion partners.

4. The method according to claim 3, wherein the probes are covalently secured on a support.

5. The method according to claim 3, wherein the detecting steps of the PCR products is based on the chip DNA technique and comprises the use of oligonucleotidic probes or cDNA secured on a miniaturized support.

6. The method according to claim 1 or 2, wherein the PCR products are denaturated with labelled probes in solution.

7. The method according to any one of claims 1 to 6, wherein to obtain cDNA, sequences including a cassette of about 40 to 60 nucleotides and 10 to 20 T patterns on one end or a random repeat of nucleotide pattern, are used as primers.

8. The method according to any one of claims 1 to 7, comprising the following steps:
- the genomic DNA or RNA, extracted from the sample cells under investigation, are subjected to the action of a compound capable of cleaving or blocking specifically the DNA or RNA of the gene, the fusion of which is under investigation,
- the PCR step(s) is (are) performed and the products so obtained are allowed to react on the one hand with two specific probes of the gene under investigation, one upstream the break points and one downstream and on the hand, probes prepared from known partner genes,
where a positive detection with the upstream probe and a negative detection on downstream probe in the first case, leading to conclude to a rearrangement of the relevant gene, and a negative conclusion in the second case, to the absence of detection of a known fusion product, or, alternatively
- the PCR products are allowed to react with a plurality of probes secured on a miniaturized support, the probe PCR products, specific hybridization highlighting the partner of the fusion gene.

9. The method according to any one of the claims 1 to 8, **characterized in that** it is applied to the detection of leukemia.

10. The method according to any one of the claims 1 to 8, **characterized in that** it is applied to the detection of translocations involving the MLL gene, and includes:
- the RT synthesis of a cDNA pool from the RNA extracted from the sample under investigation, using primers which include a cassette of 30 to 35 nucleotides complemented by a sequence of 6 or 9 random nucleotide patterns,
- the PCR amplification using a first primer located on the MLL exon 5 as specific sense primer, the 3' primer being complementary to the known oligonucleotide sequence used in the RT step, optionally followed by a further PCR amplification using an internal sense primer with respect to the first one.

11. A method according to claim 8, wherein the detection of fusion transcripts, if any, includes putting a specific probe of known MLL fusion partners into contact with denatured PCR products marked by biotine during amplification, in such conditions as will allow hybridization to occur where complementary bases are present, the probe being covalently secured on a support by coupling with streptavidine.

12. The method according to claim 11, wherein to detect new MLL-partner gene associations, total RNAs extracted from a patient are subjected to the action of ribozymes MLL- gene specific before RT-PCR, then the amplification products are allowed to react first with a probe corresponding to MLL exon 5 sequence on the 3' end of the primer used, then with a second MLL gene-specific probe located between the break points and ribozymes action site, and finally with known partner probes.

13. Application of the method according to any one of the claims 1 to 8, to solid tumor diagnostic.

14. Application of the method according to claim 13 to the detection of rearrangement of gene EWS in Ewing tumors.

15. Application of the method according to claim 13, to the detection of rearrangements of ERG, ETV1 or E1AF genes.

16. Kits for the in vitro diagnostic of pathologies associated to gene rearrangements to perform the method of any one of claims 1 to 12, comprising a couple of primers with a complementary probe of the nucleotidic sequence of the target gene and which binds said gene to form a complex, and a complementary random primer, as well as at least one specific probe of the fusion partner, said probe being linked to a solid support.

17. Kits according to claim 16, wherein at least one biotinylated probe is bound to a solid support by streptavidine coupled to a support.

18. Kits according to claim 17, wherein the solid support is a miniaturized support.

19. kits according to claim 18, when the support is a DNA chip.

## Patentansprüche

1. Verfahren zur *in-vitro*-Diagnostik von mit Gentransformationen verbundenen Krankheiten, das eine *anchored*-PCR-Stufe umfasst, **dadurch gekennzeichnet, dass** sie zusammen:
- eine oder mehrere asymmetrische PCR-Stufen, durch welche unspezifisch Gentransformationen hervorgerufen werden, wobei ein einziges Primerpaar verwendet wird, das von einem spezifischen Primer der Nucleotidsequenz, die dem Gen entspricht, das in der Lage ist, an einem Fusionsgen beteiligt zu sein, und einem zufälligen komplementären Primer (*anchored* Primer) gebildet wird, und
- eine auf das PCR-Produkt angewendete Detektionsstufe, die mittels für die Gentransformationen spezifischer Marker durchgeführt wird, um nur alle die Gene zu detektieren, die an einer Transformation beteiligt sind,
umfasst.

2. Verfahren nach Anspruch 1, **dadurch gekennzeichnet, dass** die in der Amplifikationsstufe verwendeten Primer 25 bis 40 Nucleotide umfassen.

3. Verfahren nach Anspruch 1 oder 2, **dadurch gekennzeichnet, dass** die PCR-Produkte für die Detektionsstufe markiert, denaturiert und anschließend mit Sonden, die für die Nucleotidsequenzen der Fusionspartner spezifisch sind, in Berührung gebracht werden.

4. Verfahren nach Anspruch 3, **dadurch gekennzeichnet, dass** die Sonden kovalent an einem Träger befestigt sind.

5. Verfahren nach Anspruch 3, **dadurch gekennzeichnet, dass** die Stufe der Detektion der PCR-Produkte auf dem DNA-Chip-Verfahren basiert und die Verwendung von Oligonucleotidsonden oder cDNA-Sonden, die an einem miniaturisierten Träger befestigt sind, umfasst.

6. Verfahren nach Anspruch 1 oder **2, dadurch gekennzeichnet, dass** die PCR-Produkte denaturiert und mit in Lösung befindlichen markierten Sonden in Berührung gebracht werden.

7. Verfahren nach einem der Ansprüche 1 bis 6, **dadurch gekennzeichnet, dass** zur Herstellung der cDNA als Primer Sequenzen verwendet werden, die eine Kassette aus etwa 40 bis 60 Nucleotiden und an einem Ende mit 10 bis 20 T-Mustern oder einer zufälligen Wiederholung eines Nucleotidmusters umfasst.

8. Verfahren nach einem der Ansprüche 1 bis 7, **dadurch gekennzeichnet, dass** es die Stufen:
- Unterwerfen der genomischen DNA oder RNA, die aus Zellen der zu untersuchenden Probe extrahiert worden ist, der Wirkung einer Verbindung, die in der Lage ist, die DNA oder RNA des Gens, dessen Fusion untersucht wird, spezifisch zu schneiden oder zu blockieren,
- Durchführen der PCR-Stufe(n) und Umsetzen der erhaltenen Produkte mit einerseits zwei Sonden, die für das zu untersuchende Gen spezifisch sind, eine stromaufwärts von der Region der Schnittpunkte und eine stromabwärts, und andererseits mit Sonden, die aus bekannten Partnergenen produziert worden sind, wobei es eine positive Detektion mit der stromaufwärtigen Sonde und eine negative Detektion mit der stromabwärtigen Sonde im ersten Fall erlaubt, auf eine Umordnung des betrachteten Gens und eine negative Schlussfolgerung im zweiten Fall auf das Fehlen der Detektion eines bekannten Fusionsproduktes zu schließen, oder dass in einer Abwandlung
- die PCR-Produkte mit einer Vielzahl von Sonden auf einem miniaturisierten Träger umgesetzt werden, wobei durch die spezifische Hybridisierung Sonde-markierte-PCR-Produkte der Partner des Fusionsgens nachgewiesen wird,
umfasst.

9. Verfahren nach einem der Ansprüche 1 bis 8, **dadurch gekennzeichnet, dass** es auf die Diagnostik der Leukämie angewendet wird.

10. Verfahren nach einem der Ansprüche 1 bis 8, **dadurch gekennzeichnet, dass** es für die Detektion von Translokationen, die das Gen MLL einschließen, angewendet wird und:
(a) die Synthese durch RT eines cDNA-Pools aus der RNA, die aus der zu untersuchenden Probe extrahiert worden ist, wobei Primer verwendet werden, die eine Kassette aus etwa 30 bis 35 Nucleotiden umfassen, die von einer Sequenz aus zufälligen 6 oder 9 Nucleotidmustern ergänzt wird, und
die Amplifikation durch PCR mittels eines ersten Primers, der sich auf dem Exon 5 von MLL befindet, als spezifischer Richtungsprimer, wobei der Primer an 3' zu der bekannten Nucleotidsequenz komplementär ist, die in der RT-Stufe verwendet wird, mit gegebenenfalls einer anschließenden erneuten Amplifikation durch PCR, wobei ein in Bezug auf den ersten Primer innerer Richtungsprimer verwendet wird,
umfasst.

11. Verfahren nach Anspruch 8, **dadurch gekennzeichnet, dass** das Auffinden der gegebenenfalls vorhandenen Fusionstranskripte das In-Berührung-Bringen einer für die bekannten Fusionspartner von MLL spezifischen Sonde mit den denaturierten PCR-Produkten, die mit Biotin während der Amplifikation markiert worden sind, unter Bedingungen, die eine Hybridisierung erlauben, wenn eine Basenkomplementarität existiert, wobei die Sonde kovalent an einem Träger durch Kopplung mit Streptavidin befestigt ist, umfasst.

12. Verfahren nach Anspruch 11, **dadurch gekennzeichnet, dass,** um neue Verknüpfungen MLL-Partnergen zu detektieren, die gesamte RNA, die von einem Patienten extrahiert worden ist, der Wirkung für das Gen MLL spezifischer Ribozyme vor der RT-PCR unterworfen wird und anschließend die Amplifikationsprodukte einerseits mit einer Sonde, die einer Sequenz des Exons 5 von MLL entspricht, an 3' des verwendeten Primers umgesetzt und anschließend mit einer zweiten Sonde, die ebenfalls für das Gen MLL spezifisch ist und sich zwischen den Schnittpunkten und der Wirkungsstelle der Ribozyme befindet, und schließlich mit bekannten Partnersonden umgesetzt werden.

13. Anwendung des Verfahrens nach einem der Ansprüche 1 bis 8 auf die Diagnostik solider Tumoren.

14. Anwendung des Verfahrens nach Anspruch 13 auf die Detektion einer Transformation des Gens EWS in Ewing-Tumoren.

15. Anwendung des Verfahrens nach Anspruch 13 auf die Detektion von Transformationen der Gene ERG, ETV1 oder E1AF.

16. Kits für die *in-vitro-*Diagnostik von Krankheiten, die mit Gentransformationen verbunden sind, für die Durchführung des Verfahrens nach einem der Ansprüche 1 bis 12, **dadurch gekennzeichnet, dass** sie ein Primerpaar, davon ein Primer, der zu der Nucleotidsequenz des Targetgens komplementär ist und an dieses Gen bindet, um einen Komplex zu bilden, und ein zufälliger komplementärer Primer, sowie mindestens eine für den Fusionspartner spezifische Sonde, die an einen festen Träger gebunden ist, umfassen.

17. Kits nach Anspruch 16, **dadurch gekennzeichnet, dass** mindestens eine biotinylisierte Sonde durch an den Träger gekoppeltes Streptavidin an einen festen Träger gebunden ist.

18. Kit nach Anspruch 17, **dadurch gekennzeichnet, dass** der feste Träger ein miniaturisierter Träger ist.

19. Kits nach Anspruch 18, **dadurch gekennzeichnet, dass** der Träger ein DNA-Chip ist.
